Europäisches Patentamt

European Patent Office

Office européen des brevets

(11) Veröffentlichungsnummer: **0 359 189**
**A2**

(12) **EUROPÄISCHE PATENTANMELDUNG**

(21) Anmeldenummer: **89116836.1**

(22) Anmeldetag: **12.09.89**

(51) Int. Cl.5: **A61K 6/10**

(30) Priorität: **12.09.88 DE 3831043**

(43) Veröffentlichungstag der Anmeldung:
**21.03.90 Patentblatt 90/12**

(84) Benannte Vertragsstaaten:
**AT BE CH DE ES FR GB IT LI NL SE**

(71) Anmelder: **ESPE Stiftung & Co Produktions-
und Vertriebs KG
Am Griesberg 2
D-8031 Seefeld(DE)**

(72) Erfinder: **Gasser, Oswald, Dr.
Höhenstrasse 10
D-8031 Seefeld(DE)**
Erfinder: **Ellrich, Klaus, Dr.
Auinger Strasse 16
D-8031 Wörthsee(DE)**

(74) Vertreter: **Abitz, Walter, Dr.-Ing. et al
Abitz, Morf, Gritschneder, Freiherr von
Wittgenstein Postfach 86 01 09
D-8000 München 86(DE)**

(54) **Alginat-Abformmaterial, Verfahren zu seiner Herstellung und Verwendung einer Calcium-und/oder Strontiumsalzlösung.**

(57) Beschrieben wird ein Alginat-Abformmaterial, enthaltend drei räumlich voneinander getrennte Bestandteile (a), (b) und (c), nämlich, bezogen auf die Gesamtmasse von (a) + (b) + (c),

(a) 10 bis 49,999 Gewichts-% alginathaltiges Pulver,

(b) 50 bis 89,999 Gewichts-% Wasser und

(c) 0,001 bis 10 Gewichts-% flüssige Zusammensetzung, enthaltend 1 bis 90 Gewichts-%, bezogen auf die Gesamtmasse von (c), gelöste Calcium- und/oder Strontiumsalze, wobei die Calcium- und/oder Strontiumionen-Konzentration in der Gesamtmasse von (b) + (c) 5 bis 100 mmol/l beträgt, dessen Herstellung sowie die Verwendung einer flüssigen Zusammensetzung als Mittel zur Abbindung von alginathaltigen Abformpulvern mit Wasser, die 1 - 90 Gewichts-% Calcium-und/oder Strontiumsalz enthält.

EP 0 359 189 A2

## Alginat-Abformmaterial, Verfahren zu seiner Herstellung und Verwendung einer Calcium- und/oder Strontiumsalzlösung

Die Erfindung betrifft ein Alginat-Abformmaterial.

Alginat-Abformmaterialien sind bereits seit langer Zeit bekannt zur Herstellung von Kieferabdrücken mit hinreichender Genauigkeit und erfreuen sich aufgrund ihrer billigen Herstellbarkeit einer weiten Verbreitung. Im allgemeinen wird hierzu dem Anwender ein alginathaltiges Pulver geliefert, das dann vom Anwender mit einer definierten Menge Wasser vermischt wird. Hierbei gehen die Reaktanten Alginsäuresalz und z.B. Calciumsalz in Lösung, reagieren miteinander und bilden ein unlösliches, elastisches Hydrogel. Neben einem löslichen Alginsäuresalz, z.B. Kalium- oder Natriumalginat, und einem mässig löslichen Calciumsalz, in der Regel Calciumsulfat, enthalten solche alginathaltigen Pulver Regulatoren zur Einstellung der Verarbeitungs- und Abbindezeit, wie Natriumphosphat oder Natriumpyrophosphat, sowie Füllstoffe, z.B. Kieselgur und komplexe Übergangsmetallfluoride, wie Kaliumhexafluorotitanat. Zur Verhinderung der Staubwirkung dieser Alginat-Pulvermischungen ist es bekannt, diese durch Zusatz von löslichen und unlöslichen organischen Verbindungen zu granulieren (z.B. EP-A 0 058 203, DE-A 34 39 884 und DE-A 34 10 923).

Die Verarbeitungs- und Abbindezeit der fertigen Abdruckmasse wird vom Hersteller durch die Mischung der Komponenten Alginat, Calciumsulfat und Natriumphosphat fest eingestellt und kann vom Anwender lediglich durch die Temperatur des Anmischwassers in gewissem Umfang verändert werden.

Aus der EP-A 0 086 062 ist es bekannt, als Verzögerer zur Verlängerung der Verarbeitungs- und Abbindezeit bestimmte Lithiumsalze einzusetzen, die der Anwender beim Anmischen der pulverförmigen Komponente mit dem Wasser zusetzen kann und damit eine gezielte verzögerte Abbindung erhält. Während die Einstellung einer kontrollierten Wassertemperatur für den Anwender nur mit grösserem Aufwand zu verwirklichen ist, birgt der Einsatz einer Verzögererkomponente bei unsachgemässer Dosierung immer die Gefahr, dass die Abformmasse bei einem Zuviel an Verzögerersubstanz nicht mehr abbindet, was insbesondere in der Zahnheilkunde nicht tolerabel erscheint.

Es ist weiterhin bekannt, dass die Abbindereaktion von Alginat-Abformmaterialien in geringem Umfang von der Härte des verwendeten Wassers abhängt (z.B. DE-A 21 31 964). Andererseits ist aus Skinners "Science of Dental Materials" (8. Ausgabe, 1982, S. 129) bekannt, dass ein Ersatz der nur mässig löslichen Calciumsalzverbindung durch eine gut lösliche, z.B. Calciumchlorid, zu einer unkontrolliert schnellen Abbindung führt und als Ergebnis kein elastisches Hydrogel, sondern eine Masse, erinnernd an "frisches Eiweiss" entsteht.

Aufgabe der Erfindung ist die Bereitstellung eines Alginat-Abformmaterials mit einstellbarer Verarbeitungs- und Abbindezeit.

Erfindungsgemäss wird diese Aufgabe gelöst durch ein Alginat-Abformmaterial, enthaltend drei räumlich voneinander getrennte Bestandteile (a), (b) und (c), nämlich, bezogen auf die Gesamtmasse von (a) + (b) + (c),

(a) 10 bis 49,999 Gewichts-% alginathaltiges Pulver,

(b) 50 bis 89,999 Gewichts-% Wasser und

(c) 0,001 bis 10 Gewichts-% flüssige Zusammensetzung, enthaltend 1 bis 90 Gewichts-%, bezogen auf die Gesamtmasse von (c), gelöste Calcium- und/oder Strontiumsalze, wobei die Calcium- und/oder Strontiumionen-Konzentration in der Gesamtmasse von (b) + (c) 5 bis 100 mmol/l beträgt.

Ein weiterer Gegenstand der Erfindung ist ein Verfahren zur Herstellung der erfindungsgemässen Alginat-Abformmaterialien durch Vermischen von Komponenten (a), (b) und (c) miteinander. Bevorzugt werden zunächst (b) und (c) miteinander gemischt und anschliessend (b) + (c) mit (a).

Die Erfindung betrifft ausserdem die Verwendung der flüssigen Zusammensetzung (c), enthaltend 1 bis 90 Gewichts-% bezogen auf die Gesamtmasse der Zusammensetzung, gelöste Calcium- und/oder Strontiumsalze, als Mittel zur Abbindung von alginathaltigen Abformpulvern mit Wasser.

Erfindungsgemäss wird der Vorteil erzielt, dass die Verarbeitungs- und Abbindezeit des Abformmaterials einstellbar ist, dieses z.B. schnell abbindet, ohne dass hierdurch die Eigenschaften der entstehenden Abformung negativ beeinflusst werden und der aus der Abformung ausgegossene Gipsstumpf oberflächlich geschädigt ist.

Da bei einer Reihe handelsüblicher Alginat-Pulvermischungen die Verarbeitungs- und Abbindezeit im Laufe der Lagerung immer länger wird, ist ein weiterer erfindungsgemäss erzielbarer Vorteil die Wiederherstellung der gewünschten Verarbeitungs- und Abbindezeit von zu lange gelagerten Alginat-Pulvermischungen.

Als Komponente (a) eingesetzt werden können alle bekannten pulverförmigen Alginat-Abformmaterialien, wie sie aus der o.g. Literatur bekannt sind. Beispiele für gut einsetzbare pulverförmige Komponenten

EP 0 359 189 A2

sind Zusammensetzungen, enthaltend 10 - 30 Gewichts-% Natrium- oder Kaliumalginat, 10- 30 Gewichts-% Calciumsulfat, 0,5 - 5 Gewichts-% Natriumphosphat sowie als Rest übliche Füllstoffe, Aromastoffe sowie Thixothropiehilfsmittel. Als Füllstoffe sind geeignet insbesondere Kieselgure, Natrium- oder Kaliumhexafluorotitanat, Zinkoxide. Als Thixothropiehilfsmittel können auch geringe Mengen an feinteiliger Kieselsäure enthalten sein, z.B. pyrogene Kieselsäure. Das erfindungsgemässe Alginat-Abformmaterial enthält vorzugsweise 20 - 40 Gewichts-% an (a).

Als Komponente (b) eingesetzt werden können destilliertes Wasser oder Leitungswasser, bevorzugt eingesetzt wird Leitungswasser. Das erfindungsgemässe Alginat-Abformmaterial enthält vorzugsweise 60 - 79,99 Gewichts-% an (b).

Das erfindungsgemässe Alginat-Abformmaterial enthält bevorzugt 0,01 - 1 Gewichts-% an (c).

Die Komponente (c) enthält bevorzugt 10 - 70 Gewichts-%, besonders bevorzugt 30 - 60 Gewichts-% gelöste Calcium-und/oder Strontiumsalze.

Als Rest von (c) wird vorzugsweise Wasser eingesetzt, enthaltend gegebenenfalls Aroma- und Farbstoffe. Als lösliche Calcium- und/oder Strontiumsalze werden vorzugsweise die leicht löslichen Halogenide, insbesondere Chloride, verwendet, ganz besonders bevorzugt ist eine Lösung von Calciumchlorid.

Die Konzentration an Calcium- und/oder Strontiumionen in (b) + (c) des erfindungsgemässen Abformmaterials beträgt bevorzugt 7 - 100 mmol/l, besonders bevorzugt 10 - 80 mmol/l.

Die Herstellung der flüssigen Zusammensetzung (c) erfolgt in einfacher Weise durch Auflösung von käuflichen Calcium-und/oder Strontiumsalzen in Wasser.

Vorzugsweise enthält (c) soviel physiologisch verträgliche Säure, dass der pH-Wert von (c) 2 - 6, besonders bevorzugt 3 - 5 beträgt, um der Ausfällung von Calcium- und/oder Strontiumcarbonat durch den $CO_2$-Gehalt der Luft vorzubeugen. Geeignete Säuren sind beispielsweise Zitronensäure, Weinsäure oder Ascorbinsäure u.ä. Idealerweise enthält die Zusammensetzung (c) soviel Calcium- und/oder Strontiumionen, dass die Verarbeitungs- und Abbindezeit der Abformmasse durch Dosierung von 1 - 5 Tropfen sich um einen Betrag von 15 - 30 Sekunden beschleunigt.

Vergleichsbeispiel

20 g Pulver einer staubfreien Alginat-Abdruckmasse, bestehend aus 11 % Kaliumalginat, 19,5 % Calciumsulfat, 3,7 % Tetranatriumdiphosphat, 2,8 % Kaliumtitanfluorid, 60 % Kieselgur, 1 % pyrogenes Siliciumdioxid und 2 % Zinkoxid, werden mit 40 ml Leitungswasser versetzt und homogen vermischt. Nach 2 Minuten tritt die Abbindung ein (23°C, 50 % Luftfeuchtigkeit). Das Material wird dann in ein Hygrophor gegeben (23°C, 100 % Luftfeuchtigkeit). Nach 5 Minuten kann aus dem Hygrophor ein voll elastisches Abformmaterial entnommen werden. Die physikalischen Eigenschaften der Masse sind in Tabelle 1 wiedergegeben.

Erfindungsgemässes Beispiel

40 ml Leitungswasser werden mit 4 Tropfen, entsprechend 140 mg, einer Lösung von 395 g Calciumchlorid und 0,5 g Zitronensäure in 856,3 g destilliertem Wasser versetzt und homogen vermischt. Diese Mischung wird mit 20 g Pulver der im Vergleichsbeispiel beschriebenen Alginat-Abdruckmasse versetzt und homogen vermischt. Die Abbindung setzt nun bereits bei 1 Minute 30 Sekunden ein und die Abformung ist nach 4 Minuten 30 Sekunden aus dem Hygrophor zu entnehmen. Die physikalischen Eigenschaften ergeben sich aus Tabelle 1.

3

TABELLE 1

| Abformmasse | Verarbeitungszeit Min. | Abbindezeit zeit Min. | bleibende* Deformation % | elastische* Verformung % | Druckfestigkeit* MPa |
|---|---|---|---|---|---|
| 1. Vergleichsbeispiel | 2 | 5 | 1,8 | 13 | 0,95 |
| 2. erfindungsgemässes Beispiel | 1,5 | 4,5 | 1,8 | 13 | 1,2 |

* gemessen nach ADA-Spezifikation Nr. 18 für Alginat-Abdruckmassen.

Ergebnis:

Mit dem erfindungsgemässen Abformmaterial lassen sich die Verarbeitungszeit und die Abbindezeit um jeweils eine halbe Minute verkürzen, ohne dass hierdurch die physikalischen Eigenschaften der Abformmasse negativ beeinflusst sind. Man erhÙt eine Masse mit erhöhter Druckfestikeit.

EP 0 359 189 A2

**Ansprüche**

1. Alginat-Abformmaterial, enthaltend drei räumlich voneinander getrennte Bestandteile (a), (b) und (c), nämlich, bezogen auf die Gesamtmasse von (a) + (b) + (c),

(a) 10 bis 49,999 Gewichts-% alginathaltiges Pulver,

(b) 50 bis 89,999 Gewichts-% Wasser und

(c) 0,001 bis 10 Gewichts-% flüssige Zusammensetzung, enthaltend 1 bis 90 Gewichts-%, bezogen auf die Gesamtmasse von (c), gelöste Calcium- und/oder Strontiumsalze,

wobei die Calcium- und/oder Strontiumionen-Konzentration in der Gesamtmasse von (b) + (c) 5 bis 100 mmol/l beträgt.

2. Abformmaterial nach Anspruch 1, dadurch gekennzeichnet, dass es (a) in einer Menge von 20 bis 40 Gewichts-% enthält.

3. Abformmaterial nach Anspruch 1 oder 2, dadurch gekennzeichnet, dass es (b) in einer Menge von 60 bis 79,99 Gewichts-% enthält.

4. Abformmaterial nach einem oder mehreren der Ansprüche 1 bis 3, dadurch gekennzeichnet, dass es (c) in einer Menge von 0,01 bis 1 Gewichts-% enthält.

5. Abformmaterial nach einem oder mehreren der Ansprüche 1 bis 4, dadurch gekennzeichnet, dass der Calcium- und/oder Strontiumsalzgehalt in (c) 10 bis 70, vorzugsweise 30 bis 60 Gewichts-% beträgt.

6. Abformmaterial nach einem oder mehreren der Ansprüche 1 bis 5, dadurch gekennzeichnet, dass die Calcium- und/oder Strontiumionenkonzentration in der Gesamtmasse von (b) + (c) 7 bis 100, insbesondere 10 bis 80 mmol/l beträgt.

7. Abformmaterial nach einem oder mehreren der Ansprüche 1 bis 6, dadurch gekennzeichnet, dass (c) soviel physiologisch verträgliche Säure enthält, dass der pH-Wert von (c) 2 bis 6, vorzugsweise 3 bis 5 beträgt.

8. Verfahren zur Herstellung des Alginat-Abformmaterials nach einem oder mehreren der Ansprüche 1 bis 7, dadurch gekennzeichnet, dass man (a), (b) und (c) miteinander vermischt.

9. Verfahren nach Anspruch 8, dadurch gekennzeichnet, dass man zunächst (b) mit (c) vermischt.

10. Verwendung einer flüssigen Zusammensetzung, enthaltend 1 bis 90 Gewichts-%, bezogen auf die Gesamtmasse der Zusammensetzung, gelöste Calcium- und/oder Strontiumsalze, als Mittel zur Abbindung von alginathaltigen Abformpulvern mit Wasser.

11. Verwendung einer flüssigen Zusammensetzung gemäss Anspruch 10, die 10 bis 70, insbesondere 30 bis 60 Gewichts-% gelöste Calcium- und/oder Strontiumsalze enthält.

12. Verwendung einer flüssigen Zusammensetzung gemäss Anspruch 10 oder 11, die soviel physiologisch verträgliche Säure enthält, dass ihr pH-Wert 2 bis 6, insbesondere 3 bis 5, beträgt.